(19) **Europäisches Patentamt / European Patent Office / Office européen des brevets**

(11) **EP 1 921 135 A1**

(12) **EUROPEAN PATENT APPLICATION**
published in accordance with Art. 153(4) EPC

(43) Date of publication:
**14.05.2008 Bulletin 2008/20**

(21) Application number: **06797340.4**

(22) Date of filing: **29.08.2006**

(51) Int Cl.:
*C12N 9/10* (2006.01)     *A23J 3/14* (2006.01)
*A23L 1/00* (2006.01)     *A23L 1/314* (2006.01)
*A23L 1/32* (2006.01)     *A23L 1/325* (2006.01)
*A23L 1/328* (2006.01)

(86) International application number:
**PCT/JP2006/317415**

(87) International publication number:
**WO 2007/026922 (08.03.2007 Gazette 2007/10)**

(84) Designated Contracting States:
**DE DK GB NL**

(30) Priority: **30.08.2005 JP 2005248771**

(71) Applicant: **Ajinomoto Co., Inc.**
**Tokyo 104-8315 (JP)**

(72) Inventors:
 • **ISHIDA, Rikiya**
  **Kawasaki-shi, Kanagawa 210-8681 (JP)**

 • **NAKAGOSHI, Hiroyuki**
  **Kawasaki-shi, Kanagawa 210-8681 (JP)**

(74) Representative: **Strehl, Peter**
  **Patentanwälte**
  **Strehl Schübel-Hopf & Partner**
  **Maximilianstrasse 54**
  **80538 München (DE)**

(54) **METHOD FOR PREPARATION OF TRANSGLUTAMINASE-CONTAINING MATTER HAVING REDUCED PROTEASE ACTIVITY**

(57) The invention provides a treatment method capable of reducing the protease present as an impurity in transglutaminase-containing products without reducing the activity of transglutaminase; a method for manufacturing transglutaminase-containing products with reduced protease activity by a treatment comprising maintaining a transglutaminase-containing product in which protease is present for 10 minutes or more but not more than 60 hours at greater than pH 9.0 but less than pH 13.0 at a temperature of 0°C or greater but less than 50°C; a transglutaminase-containing product obtained by this treatment method; a transglutaminase-containing product obtained by this treatment method; a transglutaminase formulation in which this transglutaminase-containing product is blended, and a method for manufacturing a food by adding this transglutaminase formulation.

EP 1 921 135 A1

## Description

Technical Field

The present invention relates to a transglutaminase-containing product with reduced protease activity and to a method for manufacturing the same. More particularly, the present invention relates to a method of reducing the activity of protease present as an impurity in transglutaminase-containing products. The present invention further relates to a transglutaminase formulation comprised of a transglutaminase-containing product with reduced protease activity and an additional component, and to a method for manufacturing food employing a transglutaminase formulation.

Technical Background

Transglutaminase is an enzymatic substance known to modify the physical properties of protein-containing materials. It is added directly to protein-containing materials to modify their physical properties, or is mixed with protein-containing materials, particularly gelatins and milk protein solutions, and employed in various substances as a binding composition. However, transglutaminase formulations manufactured using available transglutaminase-containing products, particularly transglutaminase-containing products derived from microbes, vary greatly in quality from lot to lot in the same manner as other enzymatic formulations.

The principal reason for the variation between lots in the effectiveness of transglutaminase formulations in protein-containing materials is thought to be variation in the transglutaminase activity of the transglutaminase-containing product. However, variation in quality remains even when transglutaminase activity is controlled, constituting a major operational problem.

In contrast to transglutaminase, which functions to crosslink and polymerize protein molecules, protease functions to degrade proteins. Since protease functions in an opposite manner from transglutaminase on proteins, the presence of protease as an impurity in products containing transglutaminase is considered undesirable in transglutaminase-containing products and transglutaminase formulations in which such products are blended as starting materials. However, there is currently no known method of selectively and efficiently deactivating or eliminating just protease activity without diminishing transglutaminase activity.

Thus, maintaining the quality of transglutaminase formulations requires the use of such countermeasures as selecting transglutaminase-containing products of low protease activity by testing and increasing the blending ratio of transglutaminase-containing product relative to the protease activity that is present. These countermeasures entail increased labor and starting material costs, greatly driving up the cost of manufacturing transglutaminase formulations.

Thus, there is a need for a technique that selectively deactivates just the protease present in transglutaminase-containing products without compromising transglutaminase activity.

Generally, methods of controlling the activity of protease in a transglutaminase-containing product include processing that selectively deactivates the protease in the transglutaminase-containing product or selectively eliminates the protease from the transglutaminase-containing product. In particular, causing protease to cease functioning by a chemical treatment, such as with inhibitors, is the basic means of addressing this problem.

Treatment for 16 hours at 37°C at pH 9.8, known to eliminate the enzyme activity of impurities such as protease and $\alpha$-amylase (Patent document 1), is a method of selectively deactivating protease without affecting a specific enzyme in the form of salt-tolerant glutaminase.

However, in contrast to this enzyme, which is known to be extremely stable with regard to alkalinity, it was previously not known whether the same alkali treatment of transglutaminase would selectively deactivate just the protease contained without compromising transglutaminase activity.

Further, the method of deactivating protease contained in lactase by $\gamma$-ray irradiation is known to reduce protease activity (Patent document 2). However, it is still unclear whether or not this radiation treatment can be applied as a means of selectively deactivating protease in transglutaminase-containing products. It is also unclear to what degree the reduction in protease activity achieved with this treatment is related to the effect of transglutaminase on the protein-containing material. Further, the use of radiation treatment is of little commercial value.

Patent document 1
Japanese Patent Application Publication No. H11-42086
Patent document 2
Japanese Patent Application Publication No. S51-76459

Disclosure of the Invention

One object of the present invention is to provide a treatment method capable of reducing the protease present in a transglutaminase-containing product without reducing transglutaminase activity.

A further object of the present invention is to provide a method for manufacturing a transglutaminase-containing product in which protease activity has been reduced by the above treatment method.

A still further object of the present invention is to provide a transglutaminase-containing product in which protease activity has been reduced by the above treatment method.

A still further object of the present invention is to provide a transglutaminase formulation in which is blended a transglutaminase-containing product the protease activity of which has been reduced by the above treatment method.

A still further object of the present invention is to provide a method for manufacturing a food employing a transglutaminase formulation in which is blended a transglutaminase-containing product the protease activity of which has been reduced by the above treatment method.

Unless specifically stated otherwise, "TG" shall be employed as an abbreviation of transglutaminase in the present invention.

The present invention covers the following specific inventions.

(1) A method for manufacturing a transglutaminase-containing product with reduced protease activity by a treatment comprising maintaining a transglutaminase-containing product in which protease is present as an impurity for 10 minutes or more but not more than 60 hours at greater than pH 9.0 but less than pH 13.0 at a temperature of 0°C or greater but less than 50°C.

(2) The method for manufacturing a transglutaminase-containing product according to (1) wherein said treatment is continued until the protease activity/transglutaminase activity in the transglutaminase-containing product reaches 0.00024 or below.

(3) The method for manufacturing a transglutaminase-containing product according to (1) or (2) wherein said treatment is conducted in a step of separating the transglutaminase-containing product from a fermentation solution in a process of manufacturing a transglutaminase-containing product by fermentation.

(4) A transglutaminase-containing product with reduced protease activity manufactured by the method for manufacturing according to any one of (1) to (3).

(5) A transglutaminase formulation comprising the transglutaminase-containing product according to (4) and an additional component.

(6) A transglutaminase formulation for binding application comprising the transglutaminase-containing product according to (4) and a protein-containing material.

(7) A transglutaminase formulation for binding application comprising the transglutaminase-containing product according to (4), collagen, and/or milk protein.

(8) A transglutaminase formulation for Surimi product comprising the transglutaminase-containing product according to (4) and at least one additional component selected from the group consisting of protein-containing materials, calcium salts, alkali salts, and excipients.

(9) A method for manufacturing a restructured food product employing the transglutaminase-containing product according to any one of (4) to (7).

(10) A method for manufacturing a restructured food product comprising: preparing an aqueous solution of the transglutaminase-containing product according to (4), mixing this aqueous solution with collagen, and/or milk protein to obtain a solution, and adding this solution to a protein-containing material.

(11) A method for manufacturing a Surimi product comprising adding the transglutaminase formulation according to any one of (4), (5), or (8) to ground fish meat.

(12) A method for manufacturing a transglutaminase-containing product with reduced protease activity by a treatment that comprises maintaining a transglutaminase-containing product in which protease is present as an impurity for 10 minutes to 60 hours at pH 9.5 to 13.0 at a temperature of 5 to 50°C.

(13) The method for manufacturing a transglutaminase-containing product according to (12) wherein said treatment is continued until the protease activity/transglutaminase activity in the transglutaminase-containing product reaches 0.00024 or below.

(14) The method for manufacturing a transglutaminase-containing product according to (12) or (13) wherein said treatment is conducted in a step of separating the transglutaminase-containing product from a fermentation solution in a process of manufacturing a transglutaminase-containing product by fermentation.

(15) A transglutaminase-containing product with reduced protease activity manufactured by the method for manufacturing according to any one of (12) to (14).

(16) A transglutaminase formulation comprising the transglutaminase-containing product according to (15).

(17) A transglutaminase formulation for binding application comprising the transglutaminase-containing product according to (15), collagen, and/or milk protein.

(18) A method for manufacturing a ground fish product comprising adding the transglutaminase formulation according to (16) or (17) to ground fish meat.

(19) A method for manufacturing a ground meat product comprising adding the transglutaminase formulation according to (16) or (17) to the ground meat of livestock and/or poultry.

(20) A method for manufacturing a vegetable protein-containing food comprising adding the transglutaminase formulation according to (16) or (17) to vegetable protein.

(21) A method for manufacturing a fish roe product comprising adding the transglutaminase formulation according to (16) or (17) to fish roe protein.

(22) A method for manufacturing a chicken egg-based food comprising adding the transglutaminase formulation according to (16) or (17) to a chicken egg product.

(23) A method for manufacturing a restructured food product comprising adding the transglutaminase formulation according to (16) or (17) to a protein-containing material.

(24) A method for manufacturing a restructured food product comprising: preparing an aqueous solution of the transglutaminase-containing product according to (15), mixing this aqueous solution, collagen, and/or milk protein to obtain a solution, and adding this solution to a protein-containing material.

(25) A method for manufacturing a stand-alone meat product comprising: preparing an aqueous solution of the transglutaminase-containing product according to (15), mixing this aqueous solution with a heterogeneous protein to prepare a pickling solution, and adding this pickling solution to a stand-alone meat product.

The protease activity of the transglutaminase-containing product obtained by applying the treatment method of the present invention is reduced to an extremely low level. Thus, there is no need for countermeasures such as screening transglutaminase-containing products with a trial production test or increasing the blending ratio to ensure the quality of the formulation. High-quality transglutaminase-containing products and transglutaminase formulations can be inexpensively manufactured.

Brief Description of the Drawings

Fig. 1 is a graph showing the relative activity of transglutaminase and protease in sample solutions when the treatment pH of the sample solution was varied.

Fig. 2 is a series of graphs showing the relative activity of transglutaminase and protease in a sample solution when the treatment time was varied at a prescribed treatment pH.

Fig. 3 is a graph showing the relative activity of transglutaminase and protease in a sample solution when the processing temperature was varied at a prescribed treatment pH.

Fig. 4 is a graph comparing binding strength when a transglutaminase-containing product with high protease activity was treated at a prescribed treatment pH and when such treatment was not conducted.

Fig. 5 is a graph comparing Surimi product properties when a transglutaminase-containing product with high protease activity was treated at a prescribed treatment pH of pH 11 and when such treatment was not conducted.

Best Mode for Carrying out the Invention

Implementation modes of the present invention are described below.

Although protease is known to be present as an impurity in transglutaminase-containing products, it is difficult to selectively deactivate just the protease contained without compromising the activity of the transglutaminase.

The present inventors conducted extensive research into methods of reducing the activity of protease present as an impurity in transglutaminase-containing products, resulting in the development of the above-described treatment method. Transglutaminase-containing products with low protease activity manufactured by this treatment method can be used to inexpensively manufacture high-quality transglutaminase formulations.

In the present invention, the term "protease" refers to enzyme-degrading proteins that are present either due to migration from starting materials in the course of manufacturing a transglutaminase-containing product or through secretion by microbes.

In the present invention, the term "transglutaminase" refers to a type of transferase that catalyzes an acyl transfer reaction.

The transglutaminase employed in the present invention may be derived from tissue or from microbes. However, microbial transglutaminase is desirable because of its low cost. The transglutaminase employed in the present invention may be calcium-dependent or calcium-independent. Calcium-independent transglutaminase is desirable from the perspective of having an effect that is independent of the calcium concentration of the material to which it is added.

In the present invention, the term "transglutaminase-containing product" means a liquid, or powder obtained by drying such a liquid, that is obtained from organic tissue or a microbial culture solution containing transglutaminase and then subjected to filtration and purification steps as needed. Excipients and stabilizing agents may be admixed to render the transglutaminase activity uniform or stabilize enzymatic activity.

In the present invention, the term "transglutaminase formulation" refers to a mixture of a transglutaminase-containing product and one or more additional components selected based on the objective. Here, the term "additional component" means a protein-containing-material, salt, sugar, excipient, or the like.

In the present invention, the "protein-containing material" to which the transglutaminase-containing product or transglutaminase formulation is added is a material containing a protein comprising glutamine residues serving as substrate for transglutaminase, and may be edible or inedible. Examples of edible protein-containing materials are materials derived from vegetable proteins, animal proteins, microbial proteins, and algal proteins. Examples of vegetable proteins are soy protein, wheat protein, and pea protein. Examples of animal proteins are livestock meat, poultry, fish, chicken eggs, milk, isolated purified products thereof, fish roe, blood plasma protein, gelatin, and collagen.

Calcium lactate, calcium carbonate, calcinated calcium, trisodium phosphate, sodium carbonate, and the like are readily employed salts. Examples of sugars suitable for use include sugars, starches, dextrin, and sugar alcohols. The above sugars can be employed as excipients. Dextrin, starches, lactose, and the like, which have little effect on flavor, are particularly desirable.

In the present invention, the transglutaminase-containing product is desirably treated under conditions of a pH range of greater than pH 9.0 but less than pH 13.0, with pH 9.5 or above and pH 12.5 or below being preferred and pH 10.5 or above and pH 12.5 or below being of even greater preference. This range may also be pH 9.5 to 13. During treatment, the temperature and processing times are not specifically limited so long as the transglutaminase is not deactivated. Generally, a temperature of 0°C or above and less than 50°C, preferably 10°C or above and 40°C or below, and a treatment period of 10 minutes or more and 60 hours or less, preferably 10 minutes or more and 18 hours or less, are employed. A temperature of 0 to 50°C, preferably 15 to 40°C, is also acceptable.

In the present invention, the transglutaminase-containing product is desirably treated so that the ratio of protease activity/transglutaminase activity is 0.00024 or less, preferably 0.00017 or less. The minimum practical binding strength has been determined by experience to be 50 g, with 100 g being preferred; the above activity ratios correspond to these binding strength, respectively.

The above treatment can be conducted as a step of separating the transglutaminase from a fermentation solution in a process of manufacturing a transglutaminase-containing product by fermentation. The same effect can be achieved by treating a solution obtained by dissolving in solvent a transglutaminase-containing product that has been pulverized. Further, the same effect can be achieved by treating a solution obtained by mixing a transglutaminase-containing product and an additional component (protein-containing material, salt, sugar, excipient, or the like) selected based on the objective.

In the present invention, the term "transglutaminase formulation for binding application" means a mixed powder of transglutaminase-containing product and protein-containing product, or a set provided in the form of the two in separate packages. The mixed powder may be used as is, or water may be added to obtain an aqueous solution which is then either sprinkled or spread on the object being binded, or mixed with the object being binded for use. When provided as a set, the two powders may be mixed immediately prior to use and employed in the same manner as a mixed powder, or a transglutaminase-containing product and protein-containing material may be dissolved in water and the solution coated on or mixed with the objects being binded. To the extent that storage stability adequate for product handling and distribution is achieved, the form of the formulation is not limited to that of a powder and liquid formulations may be employed.

The protein-containing material blended into a formulation for binding application may be selected from among the above-described protein-containing materials. Milk protein comprised primarily of casein, highly water-soluble gelatins, and blood plasma protein comprised primarily of fibrin all make good additional components. Among gelatins, fish gelatin is particularly desirable. Transglutaminase formulation for binding applications containing additional components in the form of gelatins afford extremely good binding strength. However, they tend to be relatively easily affected by protease. Thus, the application of the present invention affords a marked improvement in quality.

In the present invention, the term "transglutaminase formulation for Surimi product "refers to a formulation characterized by comprising a transglutaminase-containing product and at least one additional component selected from among the group consisting of protein-containing materials, calcium salts, alkali salts, and excipients.

The calcium salt may be any salt containing calcium, such as calcium lactate, calcium carbonate, hydrogen calcium phosphate, calcinated calcium, eggshell calcium, and seashell calcium. Any alkali salt that raises the pH of ground fish may be employed, readily employed examples of which are trisodium phosphate, sodium carbonate, hydrogen sodium carbonate, and calcified carbonate. Examples of protein-containing materials that are blended into transglutaminase formulation for Surimi product are sodium casein, potassium casein, soy protein, and wheat protein. Excipients in the form of sugars, starches, dextrin, sugar-alcohols, and the like may be employed. Dextrin, starches, lactose, and the like, which have little effect on flavor, are particularly desirable.

The transglutaminase-containing product and transglutaminase formulation of the present invention may be employed in restructured food products and Surimi products. They may be added to ground fish, the ground meat of livestock and poultry, gelatin, vegetable protein, fish roe, chicken egg products, and the like. The transglutaminase-containing product can also be prepared as an aqueous solution, the aqueous solution mixed with a homogeneous protein to obtain a pickling solution, and the pickling solution added to a stand-alone meat product for use in manufacturing stand-alone meat products.

In the present invention, the term "restructured food product" means a food that is obtained by bonding food starting materials. Here, the term "food starting material" means not only meats such as beef, pork, horse meat, mutton, goat meat, poultry, and chicken, but also various fish; shellfish; shrimp, crab, and other crustaceans, squid, octopus, and other mollusks; and caviar, salmon roe, cod roe, herring roe, and other fish roes. Vegetables, fruits, and the like are also included among food starting materials. Starting materials other than those given may also be employed, and two or more starting materials may be combined for use.

In the present invention, the term "unit of protease activity" is defined as follows: one unit (1U) is the amount of enzyme required to impart an increase in the colored substance in a nonprotein Lowry test solution by an amount equivalent to 1 mg of bovine serum albumin (BSA) in one minute. Since protease activity varies with measurement conditions, only values measured under identical measurement conditions can be directly compared.

The activity unit of transglutaminase in the present invention is measured and defined as follows: TG is reacted in a reaction system with a substrate in the form of benzylcarbonyl-L-glutamylglycine and hydroxylamine in pH 6.0 tris buffer solution at 37°C; the hydroxamic acid produced is used to form an iron complex in the presence of trichloroacetic acid; absorbance is measured at 525 nm; the quantity of hydroxamic acid is obtained from a calibration curve; and the quantity of enzyme required to generate $1\mu$ mole of hydroxamic acid per minute is defined as one unit (1U) (see Japanese Patent Application Publication No. S64-27471).

Embodiments

The present invention is described in detail below through embodiments. However, the present invention is in no way limited by the embodiments.

Embodiment 1

(Method of treating transglutaminase)

A 1 g quantity of sample transglutaminase (product name: Activa TG, made by Ajinomoto Corp.) was dissolved in 20 g of distilled water and 0.4 g quantities of this aqueous solution were admixed to 1.6 g of 0.1 M GTA buffer that had been adjusted to pH 3, 4, 5, 6, 7, 8, 9, 10, 11, and 12. Each of the aqueous solutions was maintained for 30 minutes at 20°C to obtain sample solutions.

(Measurement of transglutaminase activity)

pH 6.0 Tris-HCl buffer was added to the sample solutions prepared under the above-described conditions to dilute them to 0.2 percent sample transglutaminase (product name: Activa TG, made by Ajinomoto Corp.). Subsequently, transglutaminase-containing product was reacted in a reaction system with a substrate in the form of benzylcarbonyl-L-glutamylglycine and hydroxylamine at 37°C. The hydroxamic acid produced was converted to iron complex in the presence of trichloroacetic acid. Next, the absorbance of the reaction system was measured at 525 nm and the quantity of hydroxamic acid was obtained from a calibration curve. The quantity of enzyme required to generate 1 $\mu$mole of hydroxamic acid per minute was adopted as the activity unit, that is, one unit (1U).

(Measurement of protease activity)

A 2 percent KC1, 1 percent Triton X-100, and 50 mM phosphate buffer solution (pH 6.0) was added to sample solutions prepared as set forth above to dilute them to 0.5 percent sample transglutaminase (product name: Activa TG, made by Ajinomoto Corp.). The mixture was stirred with a stirrer for 60 minutes and centrifuged (3,000 rpm, 10 minutes, 20°C). The supernatant was passed through a 0.45 $\mu$m syringe filter (prepared at time of use).

A 2.5 g quantity of dimethylcasein (Sigma C9801, Casein, N,N-dimethylated from bovine milk) was precisely weighed out, 100 mL of 50 mM phosphate buffer (pH 6.0) was added, and the mixture was dissolved by stirring for 30 minutes or more at ordinary temperature (prepared at time of use).

Alkali copper test solution: 2 Percent $Na_2CO_3$ in 0.1 M NaOH, 2 percent sodium tartrate solution, and 1 percent copper sulfate pentahydrate were admixed in a 50:1:1 ratio (prepared at time of use).

Two-fold diluted phenol test solution (phenol reagent made by Wako Junyaku) and distilled water were admixed in a 1:1 ratio by volume (prepared at time of use).

Sample: Dimethylcasein solution was cooled in advance in a thermostatic vessel set to 5°C ± 0.5°C (the temperature was confirmed by a standard temperature gage). A 0.4 mL quantity of sample solution was weighed out and charged to a test tube. The test tube was placed for about 10 minutes or more in a thermostatic vessel, after which 2 mL of dimethylcasein solution was added. The mixture was immediately stirred by shaking and left standing for 24 hours at precisely 5°C ± 0.5°C. When the reaction had ended, 2 mL of 5 percent trichloroacetic acid was added and the mixture was immediately stirred. The mixture was restored to room temperature. The mixture was charged to a thermostatic vessel set to 37°C ± 0.5°C, left standing for 30 to 45 minutes, centrifuged (3,000 rpm, 10 minutes, 20°C), and passed through a 0.45 μm syringe filter. The filtrate was recovered.

Blank: A 2 mL quantity of dimethylcasein solution was charged to a test tube and left standing for 24 hours at 5°C ± 0.5°C. A 2 mL quantity of 5 percent trichloroacetic acid was added. The mixture was immediately stirred and restored to room temperature. A 0.4 mL quantity of each of the sample solutions was added and the mixtures were immediately stirred. The mixtures were placed in a thermostatic vessel set to 37°C ± 0.5°C, left standing for 30 to 45 minutes, centrifuged (3,000 rpm, 10 minutes, 20°C), and passed through 0.45 μm syringe filters. The filtrates was recovered.

A 0.4 mL quantity of filtrate was weighed out and charged to a test tube and 2 mL of alkali copper test solution was added. The mixture was stirred with shaking and left standing for 10 minutes at room temperature. Next, 0.2 mL of phenol reagent (made by Wako Junyaku) that had been diluted two-fold with water was added and the mixture was immediately stirred. Next, the mixture was placed in a thermostatic vessel set to 37°C ± 0.5°C, left standing for 30 minutes, and cooled to room temperature. The absorbance (the absorbance of the sample was denoted as A1 and that of the blank as A2) at a wavelength of 700 nm was measured for the sample and water as control.

Commercial standard purified bovine serum albumin (BSA) solution (Bio-Rad Protein Assay Standard II, known concentration) was repeatedly diluted two-fold (1-> 2) with distilled water to prepare two-fold, four-fold, and eight-fold diluted solutions (for example, when the concentration of the BSA solution was 1.2 mg/mL, the concentrations of the diluted solutions were 0.6, 0.3, 0.15 mg/mL). A 0.4 mL quantity of diluted BSA solution was weighed out and charged to a test tube, 2 mL of alkali copper test solution was added, the components were mixed by stirring, and the mixture was left standing for 10 minutes at room temperature. Next, 0.2 mL of phenol reagent diluted two-fold with water was added and the mixture was immediately stirred. The mixture was placed in a thermostatic vessel set to 37°C ± 0.5°C, left standing for 30 minutes, and then cooled to room temperature. The absorbance (S1) of this solution was measured at a wavelength of 700 nm employing water as control. Separately, distilled water was employed instead of BSA solution, the same operations were conducted, and the absorbance (SO) was measured. Employing the concentrations of various standard protein solutions and absorbance differences obtained by subtracting S0 from S1, a scatter diagram was prepared with the Microsoft software application Excel, an approximation curve was prepared as a second degree polynomial approximation, and a mathematical equation was obtained. Values obtained by subtracting S0 from absorbances A1 and A2 were substituted into the equation to calculate the protein concentrations (PA1, PA2).

The quantity of enzyme that imparted an increase in the colored substance in nonprotein Lowry test solution by an amount equivalent to 1 mg of bovine serum albumin (BSA) in one minute was defined as one unit (1U) and calculated based on the following equation.

$$\text{Protease activity (U/g)} = (PA1 - PA2) \times 4.4 \div 0.4 \div T \times V \div W$$

The symbols in the equation denote the following:

| | |
|---|---|
| PA1 | Protein concentration of the enzyme reaction solution (mg BSA/mL) |
| PA2 | Protein concentration of blank (mg BSA/mL) |
| 4.4 ÷ 0.4 | Coefficient of conversion to total solution quantity at end of reaction |
| T | Reaction time (minutes) |
| V | Dissolved volume of powder sample (mL) |
| W | Quantity of powder sample employed (g) |

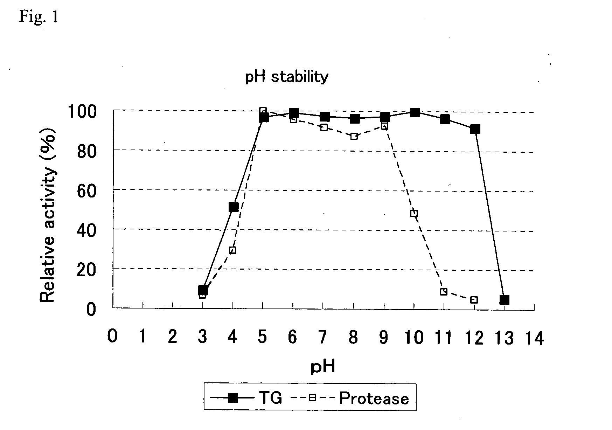

The transglutaminase activity and protease activity of the sample solutions treated under various conditions have been collected in Fig. 1 as relative values for an activity value of 100 percent when pH treatment was not conducted.

At pH 3 to 9, the relative activity value of transglutaminase and the relative activity value of protease (the activity relative to the activity of transglutaminase prior to pH treatment, defined as 100 percent) were nearly equivalent. That is, it was clear that treatment within this pH range could not reduce protease activity without reducing transglutaminase activity (Fig. 1). However, when treatment was conducted at greater than pH 9, the relative activity of protease clearly decreased more than the relative activity of transglutaminase. At pH 13 and above, the activity of transglutaminase was also seen to drop sharply. Thus, treatment of the transglutaminase solution at greater than pH 9.0 but less than pH 13.0 reduced protease activity without reducing transglutaminase activity, permitting the manufacturing of higher-quality transglutaminase.

Embodiment 2

(Method of treating transglutaminase)

A 1 g quantity of sample transglutaminase (product name: Activa TG, made by Ajinomoto Corp.) was dissolved in 20 g of distilled water and 0.4 g quantities of this aqueous solution were admixed to 1.6 g of 0.1 M GTA buffer that had been adjusted to pH 9, 10, 11, and 12. Each of the aqueous solutions was maintained for from 30 minutes to six hours at 20°C to obtain sample solutions.

(Method of Measuring transglutaminase activity)

Transglutaminase activity was measured in accordance with the method described in Embodiment 1.

(Method of measuring protease activity)

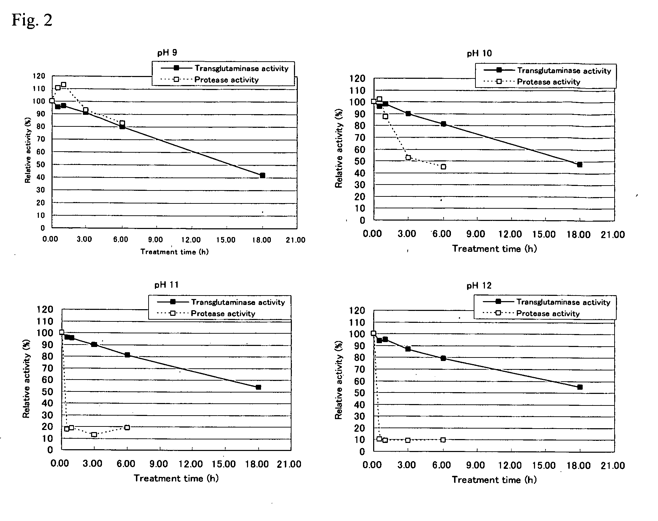

Protease activity was measured in accordance with the method described in Embodiment 1.
In the same manner as for the results of Embodiment 1, in contrast to no difference being found between the relative activity of transglutaminase and the relative activity of protease when treated for six hours at pH 9, the relative activity of protease was determined to be lower than the relative activity of transglutaminase when treated at greater than pH 9 (Fig. 2). Further, for treatment lasting 30 minutes or more at pH 11 and above, 80 percent of the relative activity of transglutaminase was maintained, while the relative activity of protease was found to drop to 20 percent or less. For treatment for 30 minutes or more at pH 12 and above, 80 percent of the relative activity of transglutaminase was maintained, while the relative activity of protease was found to drop to 10 percent or less.

Embodiment 3

(Method of treating transglutaminase)

A 1 g quantity of sample transglutaminase (product name: Activa TG, made by Ajinomoto Corp.) was dissolved in 20 g of distilled water and 0.4 g quantities of this aqueous solution were admixed to 1.6 g of 0.1 M GTA buffer that had been adjusted to pH 11. Individual aqueous solutions were maintained for 30 minutes at temperatures of 10, 20, 30, 40, and 50°C to obtain sample solutions.

(Method of measuring transglutaminase activity)

Transglutaminase activity was measured in accordance with the method described in Embodiment 1.

(Method of measuring protease activity)

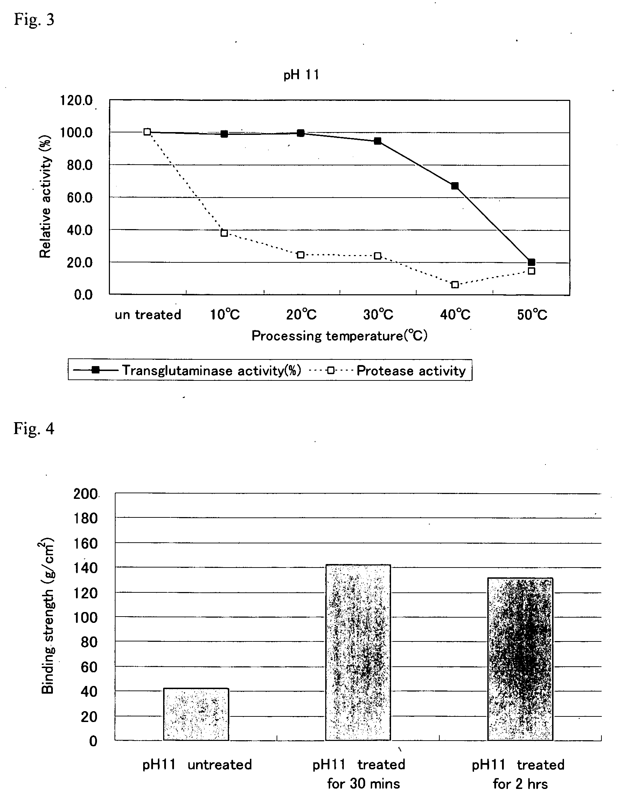

Protease activity was measured in accordance with the method described in Embodiment 1.
A reduction in the activity of protease was found to occur when the transglutaminase aqueous solutions were maintained in various temperature zones (Fig. 3). However, since the drop in activity of transglutaminase was also marked when maintained at 50°C, it was determined that protease activity could be decreased while keeping transglutaminase activity intact when the solution was maintained at less than 50°C.

Embodiment 4

An 18.0 g quantity of sample transglutaminase (product name: Activa TG, made by Ajinomoto Corp.) with a ratio of protease activity/transglutaminase activity of greater than 0.00024 and 1.2 g of fish collagen (made by Kenny & Ross Ltd.) were dissolved in 12 g of water to obtain an aqueous solution of transglutaminase fish collagen.
A 300 g quantity of small pieces of pork round meat (about 2 cm square) was admixed to the aqueous solution of transglutaminase fish collagen that had been prepared and 0.3 g of trisodium phosphate anhydride was added. The mixture was mixed and then stuffed into a casing tube with a folded width of 75 mm.

In another test area, 0.3 g of trisodium phosphate anhydride was added to a transglutaminase fish collagen solution prepared in the manner set forth above. These pH 11 aqueous solutions were maintained at 25°C for 30 minutes and 2 hours, respectively. A 300 g quantity of small pieces (about 2 cm square) of pork round meat was then added to each of the aqueous solutions, and the mixtures were mixed and stuffed into casing tubes with a folded width of 75 mm.

The mixtures of pork round meat and aqueous solutions of transglutaminase, fish collagen stuffed into casing tubes as set forth above were left standing at 5°C for 2 hours to allow the transglutaminase reaction to progress. Subsequently, the products were placed in a freezer at -40°C and stored frozen until evaluation. The frozen mixtures of pork round meat and aqueous solutions of transglutaminase fish collagen were sliced into pieces 9 mm thick and 2.5 mm in width and thawed. The binding strength was then measured by a tensile test with a texture analyzer made by Stable Micro Systems Corp.

The minimum practical binding strength has been determined by experience to be 50 $g/cm^2$. Based on the results, the binding strength, when an aqueous solution of transglutaminase fish collagen that had not been treated at pH 11 was employed, was less than 50 $g/cm^2$ (Fig. 4). However, the binding strength, when the same sample transglutaminase was treated with a pH 11 aqueous solution for 30 minutes and 2 hours, was found to be 50 $g/cm^2$ or more. The protease activity in the sample transglutaminase in which practical strength was achieved was thought to have been decreased without lowering transglutaminase activity by treatment with pH 11 aqueous solution.

Embodiment 5

Test transglutaminase with a ratio of protease activity/transglutaminase activity of greater than 0.00024 (high protease TG) and sample transglutaminase with a ratio of 0.00024 or less (low protease TG) (product name: Activa TG, made by Ajinomoto Corp.) were added in quantities of 0.336 g each to 40 mL of 1 percent brine and dissolved. A 5 mL quantity of 1 percent trisodium phosphate aqueous solution was added to the transglutaminase aqueous solution. The mixture was adjusted to pH 11 and maintained for 30 minutes at 25°C. Subsequently, 5 mL of 0.4 percent fumaric acid aqueous solution was added and neutralized to pH 7 to obtain a pH 11-treated transglutaminase-containing product. Further, an aqueous solution obtained by mixing in advance 5 mL of 1 percent trisodium phosphate aqueous solution and 5 mL of 0.4 percent fumaric acid aqueous solution was admixed with the transglutaminase aqueous solution for use as a control. Employing the transglutaminase-containing products treated as set forth above, boiled fish paste was prepared by the following procedure.

A 1,000 g quantity of flaked frozen ground fish (Alaska pollack, FA grade) was cut until the temperature reached -2 to 0°C with a Stephan cutter. A 30 g quantity of table salt was added, 350 g of ice water was added, and cutting was conducted until the temperature reached 7 to 8°C with the Stephan cutter. To this were added 20 g of granulated sugar, 40 g of potato starch, 350 g of ice water, and 2 g of Surimi product formulation and cutting was conducted until the temperature reached 7 to 8°C. The material thus obtained was stuffed into a cylindrical vinylidene chloride casing 30 mm in diameter, heated for 30 minutes in 40°C steam and then for 20 minutes in 85°C steam, and cooled by immersion in ice water.

A breaking test was conducted with a texture analyzer. Cylindrical pieces of boiled fish paste were cut to heights of 30 mm, a spherical plunger 5 mm in diameter was pressed at a rate of 1 mm/s into the center of the cross-section of the boiled fish paste, and the stress (breaking stress) at the moment of breaking was measured. Correlation coefficients for the boiled fish paste breaking stress and the protease activity under various conditions of the original sample transglutaminase powder were obtained using the Microsoft application Excel.

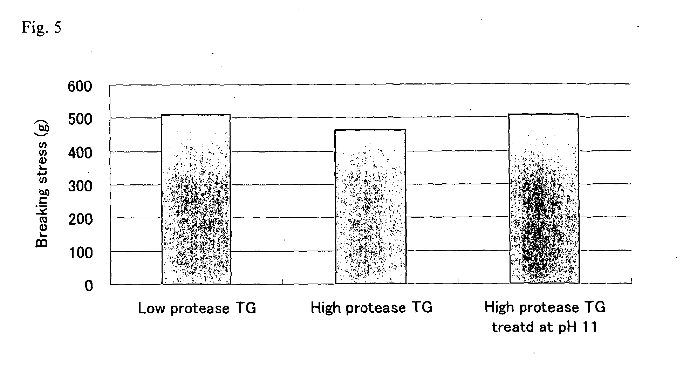

Based on experience, a difference in taste is experienced when the difference in the breaking strength of boiled fish paste is 5 percent or greater. The results are given in Fig. 5. Based on Fig. 5, the breaking stress of the high protease TG boiled fish paste when the breaking stress of low protease TG boiled fish paste was adopted as 100 percent (511.0 g) was 90.8 percent (464.2 g) and a difference in taste was found.

When the high protease TG was maintained for 30 minutes at pH 11, the breaking stress of the boiled fish paste became 99.6 percent (509.1 g), a strength at which no difference in taste was found. It was thought that by maintaining the high protease activity transglutaminase-containing product in a pH 11 aqueous solution, the protease activity was reduced without reducing the transglutaminase activity, yielding results equivalent to those obtained for low protease activity transglutaminase-containing products.

Industrial Applicability

As set forth in detail above, the method of the present invention permits the manufacturing of higher quality transglutaminase formulations by permitting a selective reduction in protease activity without reducing transglutaminase activity. It inexpensively supplies high-quality transglutaminase-containing products that are useful as additives for enhancing the quality of various materials containing proteins which have glutamine residues serving as substrates for transglutaminase, particularly components of formulations for binding application and formulations for Surimi products.

Thus, these transglutaminase-containing products expand use of the above protein-containing materials into fields beyond the area of food.

The present application is based on a Patent Application No. 2005-248771 filed in Japan, the contents of which are incorporated in full herein by this reference.

**Claims**

1. A method for manufacturing a transglutaminase-containing product with reduced protease activity by a treatment comprising maintaining a transglutaminase-containing product in which protease is present as an impurity for 10 minutes or more but not more than 60 hours at greater than pH 9.0 but less than pH 13.0 at a temperature of 0°C or greater but less than 50°C.

2. The method for manufacturing a transglutaminase-containing product according to claim 1 wherein said treatment is continued until the protease activity/transglutaminase activity in the transglutaminase-containing product reaches 0.00024 or below.

3. The method for manufacturing a transglutaminase-containing product according to claim 1 or 2 wherein said treatment is conducted in a step of separating the transglutaminase-containing product from a fermentation solution in a process of manufacturing a transglutaminase-containing product by fermentation.

4. A transglutaminase-containing product with reduced protease activity manufactured by the method for manufacturing according to any one of claims 1 to 3.

5. A transglutaminase formulation comprising the transglutaminase-containing product according to claim 4 and an additional component.

6. A transglutaminase formulation for binding application comprising the transglutaminase-containing product according to claim 4 and a protein-containing material.

7. A transglutaminase formulation for binding application comprising the transglutaminase-containing product according to claim 4, collagen, and/or milk protein.

8. A transglutaminase formulation for Surimi product comprising the transglutaminase-containing product according to claim 4 and at least one additional component selected from the group consisting of protein-containing materials, calcium salts, alkali salts, and excipients.

9. A method for manufacturing a restructured food product employing the transglutaminase-containing product according to any one of claim 4 to 7.

10. A method for manufacturing a restructured food product comprising: preparing an aqueous solution of the transglutaminase-containing product according to claim 4, mixing this aqueous solution with collagen, and/or milk protein to obtain a solution, and adding this solution to a protein-containing material.

11. A method for manufacturing a Surimi product comprising adding the transglutaminase formulation according to any one of claim 4, 5, or 8 to ground fish meat.

Fig. 1

pH stability

Fig. 2

Fig. 3

pH 11

Transglutaminase activity(%) · · ·□· · · Protease activity

Fig. 4

Fig. 5

<div align="center">

**INTERNATIONAL SEARCH REPORT**

</div>

| | International application No. |
|---|---|
| | PCT/JP2006/317415 |

A. CLASSIFICATION OF SUBJECT MATTER
*C12N9/10*(2006.01)i, *A23J3/14*(2006.01)i, *A23L1/00*(2006.01)i, *A23L1/314* (2006.01)i, *A23L1/32*(2006.01)i, *A23L1/325*(2006.01)i, *A23L1/328*(2006.01)i

According to International Patent Classification (IPC) or to both national classification and IPC

B. FIELDS SEARCHED

Minimum documentation searched (classification system followed by classification symbols)
C12N9/10, C12N15/00-15/90, A23J3/14, A23L1/00, A23L1/314, A23L1/32, A23L1/325, A23L1/328

Documentation searched other than minimum documentation to the extent that such documents are included in the fields searched
Jitsuyo Shinan Koho       1922-1996   Jitsuyo Shinan Toroku Koho   1996-2006
Kokai Jitsuyo Shinan Koho   1971-2006   Toroku Jitsuyo Shinan Koho   1994-2006

Electronic data base consulted during the international search (name of data base and, where practicable, search terms used)
BIOSIS/WPI(DIALOG), PubMed

C. DOCUMENTS CONSIDERED TO BE RELEVANT

| Category* | Citation of document, with indication, where appropriate, of the relevant passages | Relevant to claim No. |
|---|---|---|
| Y | WO 2004/078973 A1 (Ajinomoto Co., Inc.), 16 September, 2004 (16.09.04), Particularly, page 26, 10th line from the bottom to page 27, 7th line from the bottom & EP 1602722 A1 & US 2006/0019367 A1 | 5-11 |
| Y | JP 9-299065 A (Ajinomoto Co., Inc.), 25 November, 1997 (25.11.97), Particularly, Claims; Par. Nos. [0004], [0016] (Family: none) | 5-11 |
| Y | JP 2000-004841 A (Ajinomoto Co., Inc.), 11 January, 2000 (11.01.00), Particularly, Claims; Par. Nos. [0020], [0046] (Family: none) | 5-11 |

☒ Further documents are listed in the continuation of Box C.  ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 24 November, 2006 (24.11.06) | 05 December, 2006 (05.12.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

<table>
<tr><td colspan="2">**INTERNATIONAL SEARCH REPORT**</td><td>International application No.<br>PCT/JP2006/317415</td></tr>
</table>

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 8-00176 A  (Ajinomoto Co., Inc.),<br>26 March, 1996 (26.03.96),<br>Particularly, Claims; Par. Nos. [0019], [0037]<br>(Family: none) | 5-11 |
| Y | JP 7-327642 A  (Asama Chemical Co., Ltd.),<br>19 December, 1995 (19.12.95),<br>Particularly, Par. Nos. [0017] to [0019]<br>& EP 685164 A1          & US 5858423 A1 | 5-11 |
| P,Y | JP 2005-229807 A  (Ajinomoto Co., Inc.),<br>02 September, 2005 (02.09.05),<br>Par. Nos. [0023] to [0037]<br>(Family: none) | 5-11 |
| A | PASTERNACK R et al, Bacterial pro-<br>transglutaminase from Streptoverticillium<br>mobaraense purification, characterisation<br>and sequence of the zymogen, Eur J Biochem.,<br>1998, vol.257, no.3, p.570-576 | 1-11 |
| A | GERBER U et al, A rapid and simple method<br>for the purification of transglutaminase from<br>Streptoverticillium mobaraense, Biochem J,<br>1994, vol.299, p.825-829 | 1-11 |

Form PCT/ISA/210 (continuation of second sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H1142086 B
- JP S5176459 A
- JP S6427471 B
- JP 2005248771 A